# FASCICULE DE BREVET EUROPEEN

(11) **EP 0 705 818 B1**
(45) Date de publication et mention de la délivrance du brevet: **07.10.1998**
(21) Numéro de dépôt: 95402074.9
(22) Date de dépôt: 14.09.1995
(51) Int. Cl.: C07C 323/16, C10M 135/24

(54) **Nouveaux additifs pour compositions lubrifiantes, notamment pour des huiles de laminage de produits métallurgiques, et leurs procédés de préparation, et compositions lubrifiantes ainsi additivées**
Zusätze für Schmierölzusammensetzungen, insbesondere für Öle zum Walzen von metallischen Produkten, Verfahren zu ihrer Herstellung sowie diese Zusätze enthaltende Schmierölzusammensetzungen
Additives for lubrication compositions, notably for rolling oils for metallic products, processes for their preparation and lubricant compositions containing these additives

(30) Priorité: 29.09.1994 FR 9411608
(43) Date de publication de la demande: 10.04.1996
(73) Titulaire: SOLLAC S.A., 92800 Puteaux (FR)
(72) Inventeur: Hiver, Patricia Nathalie, F-57070 Metz Borny (FR); Delmotte, Christophe, F-60290 Rantigny (FR); Dicko, Amadou, F-57070 Metz Vallieres (FR); Paquer, Daniel André, F-54500 Vandoeuvre (FR)
(74) Mandataire: Ventavoli, Roger

(56) Documents cités:
- FR-A- 2 629 817
- US-A- 4 731 190

## Description

L'invention concerne le domaine des lubrifiants, et plus particulièrement les lubrifiants utilisés lors du laminage à froid des produits métallurgiques, notamment sidérurgiques.

Pour laminer à froid à grande vitesse un produit métallurgique tel qu'une tôle d'acier, il est nécessaire de lubrifier abondamment et de refroidir l'emprise des cylindres. Une émulsion d'huile de laminage dans de l'eau est utilisée à cet effet. L'huile a pour fonction de diminuer le frottement entre la tôle et le cylindre, et l'eau permet l'évacuation de la chaleur produite. Cette émulsion huile/eau, où la proportion d'huile est généralement de 5 à 12 %, est formée dans des cuves avoisinant le laminoir, d'où elle est extraite pour être dispersée sur la tôle. Cette émulsion doit avoir une stabilité suffisante pour conserver ses propriétés lors de son transfert entre les cuves et le laminoir.

Lorsqu'on utilise une émulsion huile-eau pure, il n'est cependant pas possible de réaliser un laminage à grande vitesse sans rencontrer des problèmes de qualité sur les produits. On constate en effet, dans ces conditions, l'apparition de défauts de surface tels que des griffes de chaleur, des rayures, des encrassements, des zones brillantes ou laiteuses... On peut assister également à une augmentation du taux de carbone résiduel (après recuit) et du fer de surface (fines de fer), et à l'apparition d'un phénomène appelé "graphitisation".

L'adjonction d'un tensio-actif à une telle émulsion permet non seulement de maintenir l'émulsion, mais aussi d'adapter la formation du film d'huile en surface de la tôle aux conditions de laminage et au type de produit laminé. Cependant, ces tensioactifs tendent à diminuer le pouvoir lubrifiant de l'émulsion.

Il a été suggéré (article "The load-carrying mechanism of organic sulfur compounds", ASLE Trans., 11, 162-175) d'ajouter à l'émulsion un additif soufré, en l'occurrence un disulfure organique symétrique (par exemple le dibenzyle disulfure), présentant des propriétés extrême-pression. On forme ainsi un film protecteur soufré à la surface du métal qui limite l'apparition des défauts.

D'autres auteurs ont envisagé la possibilité d'utiliser comme additifs des composés organiques bifonctionnalisés, l'une de ces fonctions ayant des propriétés tensio-actives et l'autre ayant des propriétés extrême-pression. Il a été ainsi proposé (article "Effect of chemical composition of additives on the antiwear and extreme pressure properties of metalworking systems", Lubrication Challenges in Metalworking and Processing, Proceedings First International Conference, Chicago, 1978, pp 99-105) l'utilisation d'esters de phosphate alkyl polyéthoxylés. La fonction phosphorée a des propriétés anti-usure et extrême-pression, alors que la chaîne polyéthoxylée a des propriétés tensio-actives. Il a été aussi proposé (article "New surfactants for soluble extreme pressure cutting oil", J. Dispersion Science and Technology, 10(3), pp 251-264) d'utiliser des alcools polyéthoxylés chlorés ou bromés.

Les additifs bifonctionnalisés qui ont été proposés jusqu'ici ne peuvent cependant être considérés comme pleinement satisfaisants. En effet, certains d'entre eux, tels que les additifs phosphorés ioniques, ne sont pas solubles dans l'huile, et peuvent apporter à l'émulsion une conductivité électrique qui joue en défaveur de sa stabilité. Quant aux additifs chlorés ou bromés, leur utilisation risque de provoquer un début de corrosion de la tôle. De plus, l'incinération des résidus d'huile contenant de tels additifs lors des traitements thermiques ultérieurs de la tôle entraînerait la formation de gaz très polluants.

Le but de l'invention est de proposer de nouveaux additifs bifonctionnalisés pour huile de laminage ayant à la fois des propriétés tensioactives, anti-usure et extrême-pression, exempts des inconvénients des additifs ioniques et non agressifs pour les produits sidérurgiques, tout en demeurant d'un coût acceptable.

A cet effet, l'invention a pour objet des composés de formule générale 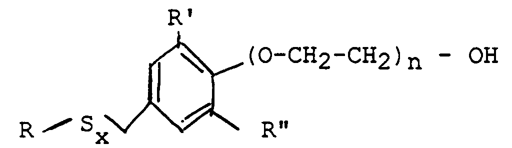 dans laquelle R est un radical alkyle, tertioalkyle, benzyle ou allyle fonctionnalisé ou non, R' et R'', semblables ou différents, sont un atome d'hydrogène ou un radical alkyle ou tertioalkyle fonctionnalisé ou non, x est un nombre de 1 à 5 et n est un nombre de 1 à 25.

L'invention a également pour objet des compositions lubrifiantes comprenant une huile de base et au moins un desdits composés, ledit composé conférant auxdites compositions lubrifiantes une teneur en soufre comprise entre 0,5 et 1,5 % en masse, et des émulsions huile-eau à 5 à 12 % en masse d'huile renfermant au moins l'un desdits composés.

L'invention a enfin pour objet des procédés de préparation desdits composés.

Comme on l'aura compris, les molécules de ces nouveaux additifs sont constituées par un noyau benzylique, substitué d'une part par un groupement polyéthoxylé, et, d'autre part, par une chaîne hydrocarbonée soufrée.

Ces molécules nouvelles comportent donc bien d'une part une fonction tensioactive assurée par le groupement polyéthoxylé et par le noyau benzylique, et d'autre part une fonction anti-usure et/ou extrême-pression assurée par la chaîne hydrocarbonée soufrée. Outre leurs bonnes performances en tant qu'additifs aux huiles de laminage, ces molécules présentent l'avantage d'être synthétisables à partir de produits de base courants et par des méthodes peu onéreuses qui seront explicitées plus loin.

Selon la formule générale: 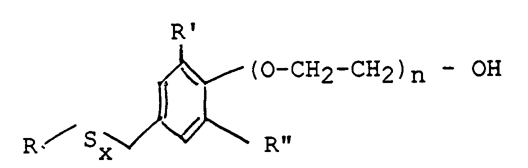 on a, par exemple, synthétisé les 15 composés figurant dans le tableau 1, où R' et R'' sont des atomes d'hydrogène (le nombre de motifs n est un nombre moyen):

**Tableau 1**

| Numéro | Groupement R | x | Motifs n |
|---|---|---|---|
| 1 | tButyle | 1 | 10 |
| 2 | tButyle | 1 | 1 |
| 3 | tButyle | 1 | 7,8 |
| 4 | tButyle | 1 | 2 |
| 5 | tButyle | 1 | 3,8 |
| 6 | Benzyle | 1 | 2 |
| 7 | Benzyle | 1 | 3,5 |
| 8 | Phényle | 1 | 1,6 |
| 9 | Nonyle | 1 | 1 |
| 10 | Dodécyle | 1 | 2,1 |
| 11 | tButyle | 2 | 1 |
| 12 | tButyle | 2 | 2 |
| 13 | tButyle | 2 | 3,8 |
| 14 | Benzyle | 2 | 3,7 |
| 15 | tDodécyle | 2 | 2,9 |

Les performances de certains de ces composés en tant qu'additifs de l'huile de laminage classique du type "Soprolamine 25" sont résumées dans le tableau 2. Les essais ont été réalisés sur des émulsions huile-eau à 10 % en masse d'huile. Dans le cas des huiles additivées selon l'invention, la proportion d'additif dans l'huile était telle qu'elle imposait à l'huile additivée une teneur de 1 % de soufre en masse. La taille des particules a été déterminée à l'aide d'un granulomètre, et les tests tribologiques ont été conduits sur une machine quatre billes selon les modalités classiques pour les essais d'huiles pures. On a opéré à des pressions voisines des pressions de laminage habituelles, de l'ordre de 15 à 30 t/cm². Par "diamètre d'usure", on entend la mesure de l'usure engendrée pendant une heure sur les trois billes inférieures sous une charge de 40 kgf. Par "soudure", on entend la charge pour laquelle, au bout de 10 sec d'essai, on observe une soudure des billes les unes aux autres.

**Tableau 2**

| émulsion | taille des particules (µm) | diamètre d'usure (mm) | charge limite avant grippage (kgf) | soudure (kgf) |
|---|---|---|---|---|
| A: eau + soprolamine 25 | 22 | 0,7 | 80 | 115 |
| B: eau + soprolamine 25 + additif 1 | 7 | 0,45 | - | 135 |
| C: eau + soprolamine 25 + additif 5 | 11,1 | 0,95 | 150 | 160 |
| D: eau + soprolamine 25 + additif 6 | 18,9 | 0,89 | 130 | 150 |

On voit d'après la taille des particules de l'émulsion B que, du point de vue de la stabilité de l'émulsion, traduite par la plus ou moins faible taille des particules, l'additif 1 est le plus performant. C'est celui pour lequel la chaîne éthoxylée est la plus longue (n = 10) et qui présente donc les propriétés tensioactives les plus poussées. On constate également que les propriétés anti-usure, traduites par le diamètre d'usure, sont améliorées par rapport à l'émulsion A renfermant de l'huile non additivée. Les additifs 5 et 6, dont les chaînes éthoxylées sont plus courtes (respectivement n = 2 et n = 3,5) diminuent les propriétés anti-usure des émulsions C et D. En revanche, on voit d'après les valeurs de la charge de soudure des billes que ces additifs 5 et 6 améliorent nettement le comportement extrême-pression des émulsions C et D par rapport aux émulsions A et B.

Ces additifs peuvent être ajoutés à l'huile de laminage avant la formation de l'émulsion dans des proportions telles qu'elles imposent à l'huile additivée une teneur de 0,5 à 1,5 % de soufre en masse. Du fait de leurs propriétés tensio-actives qui favorisent leur mélange à l'émulsion, ils peuvent également être introduits directement dans l'émulsion en cours de formation, séparément de l'huile non additivée, et dans les mêmes proportions que dans le cas précédent.

On va à présent décrire un exemple de synthèse de composés selon l'invention, à savoir les composés : 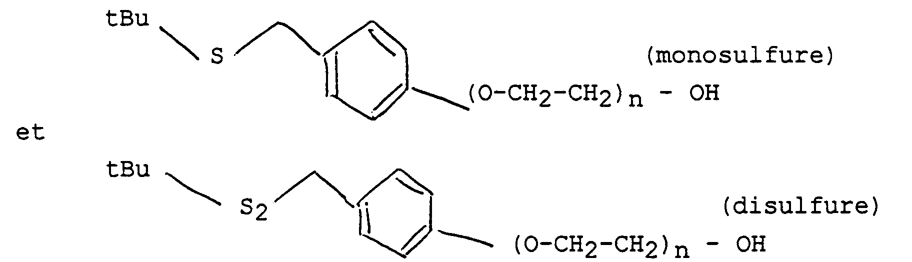 Ils sont réalisés à partir du p.crésol.

### - étape 1 : condensation des motifs polyéthoxylés

Le branchement des motifs polyéthoxylés sur le p.crésol s'effectue par polymérisation d'oxyde d'éthylène en présence d'une quantité catalytique de sodium.

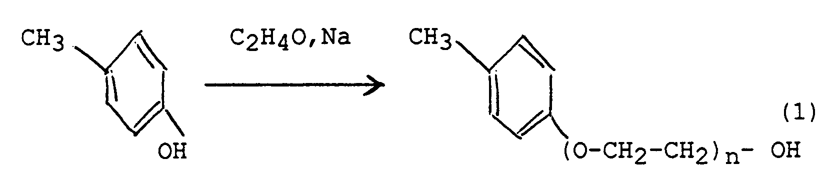

Le produit (1) est en fait un mélange statistique de produits polyéthoxylés répondant à une courbe de Gauss. n est en moyenne de 3,8 dans les conditions qui vont être décrites.

Dans un autoclave de 250 ml muni d'un manomètre, d'une arrivée d'argon, d'une arrivée d'oxyde d'éthylène, d'une agitation mécanique et d'un robinet de sortie, on introduit 10,8 g (0,1 mole) de p.crésol et 0,08 g (0,03 mole) de sodium. Le mélange est chauffé à 180°C pendant une heure. On refroidit ensuite à -5°C et on fait passer un courant d'argon. On condense ensuite la quantité d'oxyde d'éthylène voulue dans le réacteur.

L'autoclave est ensuite fermé et on applique une pression de 10 bars. Le milieu réactionnel est porté à une température de 200°C pendant trois heures. En fin de réaction, le produit (1) est acidifié par 50 ml d'une solution d'acide sulfurique à 10 %.

Le rendement de la réaction est de 100 %. La longueur de la chaîne polyéthoxylée dépend de la quantité d'oxyde d'éthylène introduite.

### - étape 2 : protection de la fonction hydroxylique

Cette étape vise à substituer de manière réversible l'hydrogène du groupement hydroxyle de (1), afin d'éviter que lors des étapes ultérieures, les réactifs ne viennent attaquer cet hydroxyle de préférence aux sites réactionnels où on désire qu'ils agissent.

La protection utilisée est l'ester benzoïque.

La condensation du chlorure de benzoyle sur le composé (1) conduit au produit protégé (2).

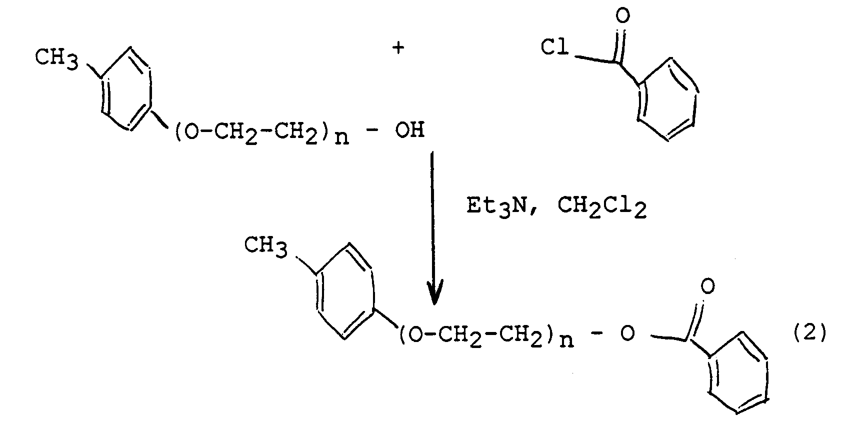

Dans un tricol de 500 ml muni d'un réfrigérant, d'une ampoule à brome et d'une agitation magnétique, introduire 0,1 mole du produit précédent (1), 100 ml de triéthylamine et 200 ml de dichlorométhane. Après homogénéisation de la solution, additionner très lentement 14 g (0,1 mole) de chlorure de benzoyle. Laisser ainsi sous agitation à température ambiante pendant 1 heure.

Le sel formé est éliminé par filtration et le solvant évaporé sous pression réduite.

Dans ces conditions, le rendement de la réaction est de 98 %. On peut noter que, de manière générale, ce type de réaction s'effectue avec des rendements de l'ordre de 70 % au moins, selon le type de subsituant fixé sur le noyau aromatique.

### - étape 3 : halogénation du méthylène aromatique

L'halogénation (ici une bromation) s'effectue par réaction radicalaire en condensant la N-bromo succinimide (NBS) sur le composé (2) dans une solution de tétrachlorure de carbone anhydre et en présence d'Azo-(bis)isobutyronitrile (AIBN) comme initiateur.

Le produit (3) est obtenu avec des rendements quantitatifs.

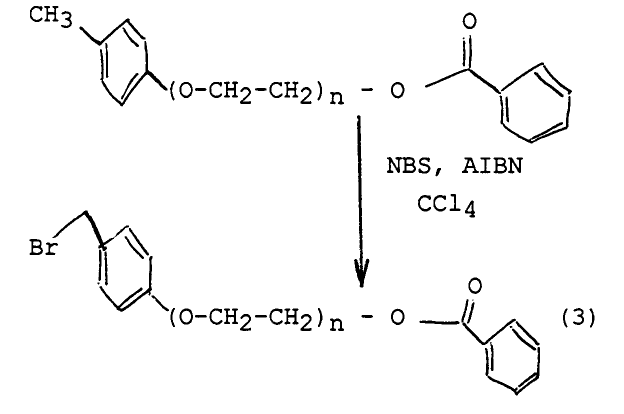

Dans un tricol de 250 ml muni d'un réfrigérant, d'une ampoule à brome et d'une agitation magnétique, dissoudre 0,07 mole du dérivé précédent (2) dans 150 ml de tétrachlorure de carbone anhydre. Additionner ensuite lentement 13 g (0,073 mole) de N-bromo succinimide et 1 g d'Azo-(bis)isobutyronitrile (AIBN). Porter ensuite à reflux pendant 2 heures.

En fin de réaction, la succinimide formée est éliminée par filtration et le solvant est évaporé sous pression réduite.

### - étape 4 : formation du sulfure

### a) Cas du monosulfure

Le monosulfure est obtenu par condensation du terbutylate de potassium sur le composé bromé précédent (3). Le sulfure (4) ainsi obtenu peut être isolé avec des rendements de l'ordre de 65 %.

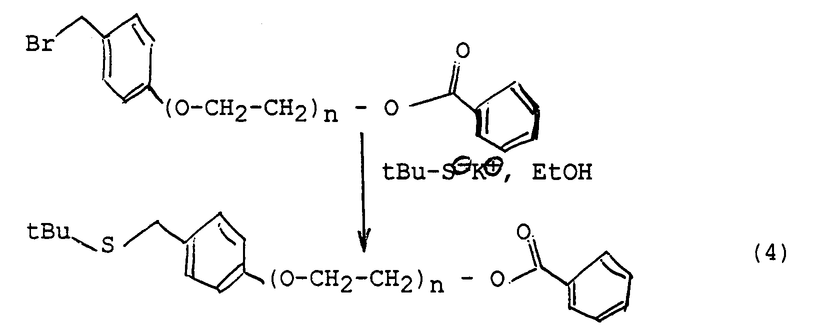

Dans un réacteur muni d'un réfrigérant, d'une ampoule à brome et équipé d'une agitation magnétique, on dissout 1,1 g (0,02 mole) de potasse dans 100 ml d'éthanol. A 50°C, on ajoute un équivalent de thiol et on maintient à cette température pendant 30 minutes. La solution est ramenée à température ambiante afin d'additionner goutte à goutte 0,02 mole du dérive halogéné (3). Le mélange est ensuite porté à reflux pendant une nuit.

Après refroidissement, le sel formé est éliminé par filtration. Le filtrat est neutralisé par de l'acide chlorhydrique dilué (10 %) puis la phase organique est extraite au dichlorométhane. Après lavage à l'eau, la phase organique est séchée sur sulfate de sodium. Le solvant est éliminé par évaporation sous vide.

### b) cas du disulfure

Pour accéder aux disulfures, dans une première étape il faut préparer un sel de Bunte par condensation du thiosulfate de sodium sur le dérivé (3). Ensuite le sel ainsi formé est additionné sur le terbutylate de potassium préalablement préparé.

Le disulfure (5) est obtenu avec un rendement de 52 %

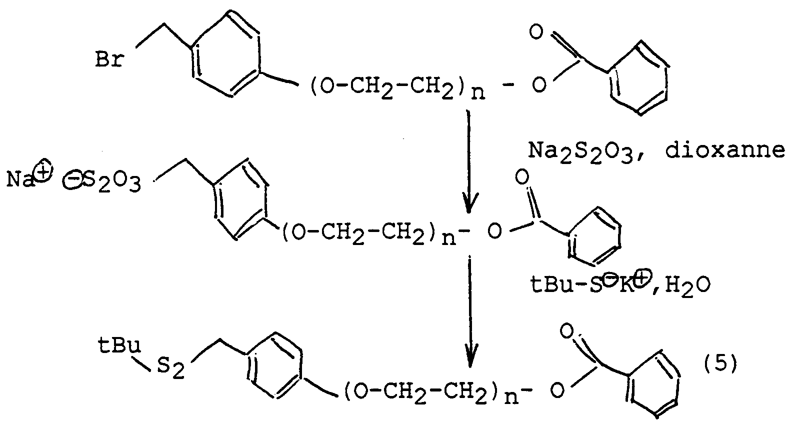

### Synthèse du sel de Bunte :

Dans un tricol de 500 ml muni d'un réfrigérant, d'une ampoule à brome et d'une agitation magnétique, introduire 5 g (0,02 mole) de thiosulfate de sodium 10 ml d'eau et 200 ml de dioxanne. Homogénéiser et additionner goutte à goutte 0,02 mole de dérivé (3). Le mélange est porté à 80°C pendant 4 heures.

### Synthèse du thiolate :

Dans un tricol de 100 ml muni d'un réfrigérant, d'une ampoule à brome et d'une agitation magnétique, introduire 1,2 g (0,02 mole) de potasse et additionner 20 ml d'eau. Additionner ensuite un équivalent de terbutyl mercaptan et porter le mélange à 50°C pendant 30 minutes.

### Synthèse du disulfure :

Additionner lentement la solution aqueuse du mercaptate dans la solution contenant l'alkyl thiosulfate. Laisser sous agitation durant 1 heure.

Après refroidissement, évaporer le solvant sous vide et laver le résidu au dichlorométhane. Sécher ensuite la phase organique sur du thiosulfate de sodium et évaporer le solvant sous vide.

### - étape 4 : déprotection de la fonction hydroxylique

La déprotection de la fonction hydroxylique est effectuée par saponification dans une solution de soude.

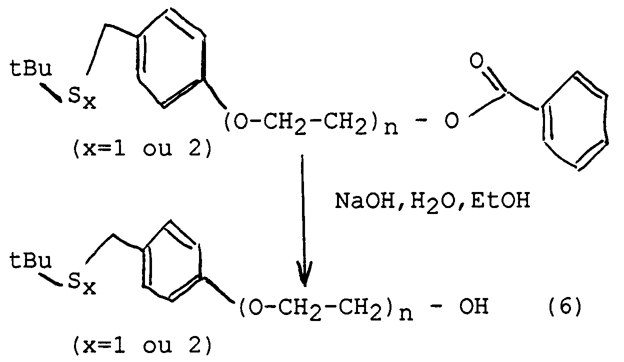

Le produit (6) est isolé avec un rendement de 67 %.

Dans un tricol de 250 ml muni d'un réfrigérant, d'une ampoule à brome et d'une agitation magnétique, introduire 1,1 g (0,02 mole) de potasse dans une solution de 50 ml de dioxanne et 50 ml d'eau. Après dissolution, additionner un équivalent de produit à déprotéger (4) ou (5). Laisser ainsi sous agitation pendant 3 heures.

Evaporer ensuite le dioxanne sous vide puis extraire le résidu au dichlorométhane. Sécher la phase organique au sulfate de sodium et évaporer le solvant sous vide.

Il est bien entendu que les synthèses qui viennent d'être décrites ne sont que des exemples susceptibles de connaître des variantes, notamment dans le choix du groupement protecteur de l'hydroxyle ou du mode de formation du sulfure.

L'une de ces variantes peut consister à débuter la synthèse par la protection de l'hydroxyle du p.crésol. On réalise ensuite la bromation du méthylène aromatique, puis la formation du sulfure, puis la déprotection de l'hydroxyle. On obtient alors un composé du type: 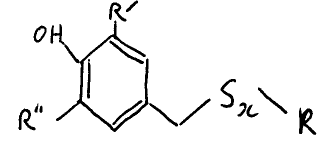 décrit dans le document FR-A-2629817. On procède enfin seulement à la condensation des motifs polyéthoxylés.

Si on désire obtenir une chaîne soufrée plus longue, on applique les méthodes de synthèse connues pour l'obtention de polysulfures.

Comme on l'a vu, ces synthèses ne mettent en jeu que des réactifs courants et bon marché, et ne comportent qu'un nombre limité d'étapes réactionnelles. Les objectifs économiques que les inventeurs s'étaient assignés sont donc atteints de manière satisfaisante.

L'invention n'est pas limitée aux composés qui viennent d'être décrits en détail. En particulier, le radical R peut être constitué par un groupement alkyle, tertioalkyl, benzyle ou allyle, éventuellement fonctionnalisé. De même, R' et R'' qui peuvent être semblables ou différents, peuvent être constitués par un atome d'hydrogène, ou un groupement alkyle ou tertioalkyle, fonctionnalisé ou non. Le nombre n d'atomes de soufre peut varier de 1 à 5, et le nombre n de groupements éthoxy de 1 à 25. Les huiles ainsi additivées sont particulièrement destinées à être utilisées pour former des émulsions huile-eau pour les opérations de laminage à froid des produits sidérurgiques, mais il va de soi que d'autres utilisations de ces huiles additivées sont envisageables, à l'état pur ou en émulsion. Il va de soi également que les additifs de l'invention peuvent être utilisés seuls ou en mélange.

## Revendications

1. Composés de formule générale 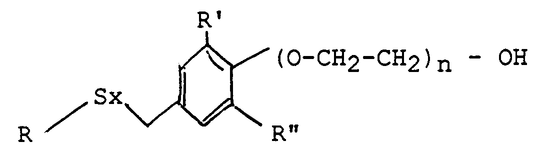 dans laquelle R est un radical alkyle, tertioalkyle, benzyle ou allyle fonctionnalisé ou non, R' et R'', semblables ou différents, sont un atome d'hydrogène ou un radical alkyle ou tertioalkyle fonctionnalisé ou non, x est un nombre de 1 à 5 et n est un nombre de 1 à 25.

2. Composés selon la revendication 1, caractérisés en ce que x est égal à 1 ou 2 et en ce que n est un nombre de 1 à 10.

3. Compositions lubrifiantes, caractérisées en ce qu'elles comprennent une huile de base et au moins un composé selon la revendication 1 ou 2, ledit composé étant présent à une concentration telle qu'elle impose à ladite composition une teneur en soufre comprise entre 0,5 et 1,5 % en masse.

4. Emulsion aqueuse de lubrification pour le laminage de produits métallurgiques, caractérisée en ce qu'elle renferme une huile de base à raison de 5 à 12 % en masse de ladite émulsion et au moins un composé selon la revendication 1 ou 2, ledit composé étant présent à une concentration telle que la teneur en soufre de ladite émulsion est comprise entre 0,5 et 1,5 % en masse de sa teneur en huile de base.

5. Procédé de synthèse d'un composé ou d'un mélange de composés selon la revendication 1, caractérisé en ce que:
- on réalise une condensation de motifs polyéthoxylés sur un composé de formule 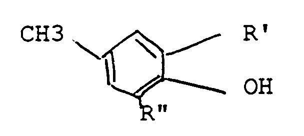 de manière à obtenir un composé intermédiaire ou un mélange de composés du type 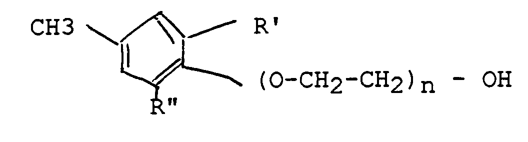
- on réalise une protection du groupement hydroxyle dudit composé intermédiaire ;
- on réalise une substitution du méthylène aromatique dudit composé intermédiaire par une chaîne soufrée du type R - Sₓ- ;
- on réalise la déprotection dudit groupement hydroxyle.

6. Procédé de synthèse d'un composé ou d'un mélange de composés selon la revendication 1, caractérisé en ce que:
- on réalise une protection du groupement hydroxyle d'un composé du type 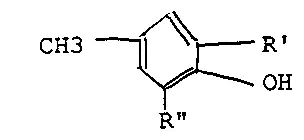
- on réalise une substitution du méthylène aromatique dudit composé par une chaîne soufrée du type R-Sₓ- ;
- on réalise la déprotection dudit groupement hydroxyle ;
- on réalise une condensation de motifs polyéthoxylés sur ledit groupement hydroxyle.

7. Procédé selon la revendication 5 ou 6, caractérisé en ce que x est égal à 1 et qu'on réalise ladite substitution du méthylène aromatique par une chaîne soufrée par halogénation dudit méthylène et condensation de R-S⁻K⁺ sur ledit méthylène halogéné.

8. Procédé selon la revendication 5 ou 6, caractérisé en ce que x est égal à 2 et qu'on réalise la substitution du méthylène aromatique par une chaîne soufrée par halogénation dudit méthylène, on prépare un sel de Bunte du composé ainsi obtenu, et on additionne ledit sel sur le composé R-S⁻K⁺ préalablement préparé.

## Claims

1. Compounds of general formula 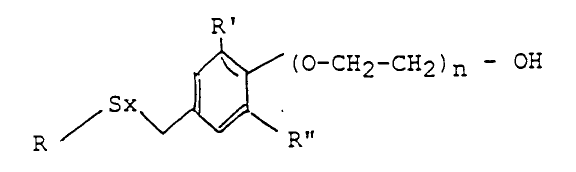 in which R is an optionally functionalized alkyl, tert-alkyl, benzyl or allyl radical, R' and R'', which are alike or different, are a hydrogen atom or an optionally functionalized alkyl or tert-alkyl radical, x is a number from 1 to 5 and n is a number from 1 to 25.

2. Compounds according to Claim 1, characterized in that x is equal to 1 or 2 and in that n is a number from 1 to 10.

3. Lubricating compositions, characterized in that they comprise a base oil and at least one compound according to Claim 1 or 2, the said compound being present at a concentration such that it imposes a sulphur content of between 0.5 and 1.5% by mass on the said composition.

4. Aqueous lubricating emulsion for the rolling of metallurgical products, characterized in that it contains a base oil in a proportion of 5 of 12% by mass of the said emulsion and at least one compound according to Claim 1 or 2, the said compound being present at a concentration such that the sulphur content in the said emulsion is between 0.5 and 1.5% by mass of its base oil content.

5. Process for the synthesis of a compound or of a mixture of compounds according to Claim 1, characterized in that:
- a compound of formula 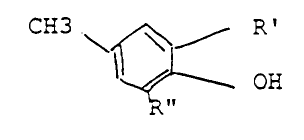 is condensed with ethylene oxide units, so as to obtain an intermediate compound or a mixture of compounds of the type 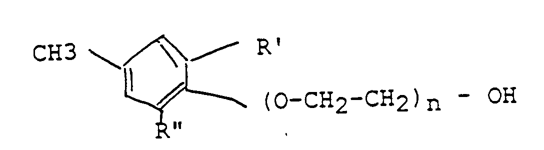
- the hydroxyl group of the said intermediate compound is protected;
- the aromatic methyl of the said intermediate compound is substituted by a sulphur-containing chain of the R-Sₓ- type;
- the said hydroxyl group is deprotected.

6. Process for the synthesis of a compound or of a mixture of compounds according to Claim 1, characterized in that:
- the hydroxyl group of a compound of the type 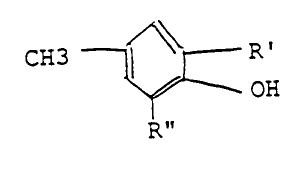 is protected;
- the aromatic methyl of the said compound is substituted by a sulphur-containing chain of the R-Sₓ-type;
- the said hydroxyl group is deprotected;
- the said hydroxyl group is condensed with ethylene oxide units.

7. Process according to Claim 5 or 6, characterized in that x is equal to 1 and that the said substitution of the aromatic methyl by a sulphur-containing chain is carried out by halogenation of the said methyl and condensation of R-S⁻K⁺ with the said halogenated methyl.

8. Process according to Claim 5 or 6, characterized in that x is equal to 2 and that the aromatic methyl is substituted by a sulphur-containing chain by halogenation of the said methyl, a Bunte salt of the compound thus obtained is prepared and the said salt is added to the preprepared compound R-S⁻K⁺.

## Patentansprüche

1. Verbindungen der allgemeinen Formel 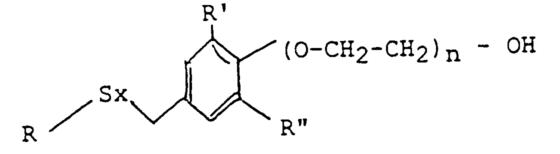 in der R ein funktionalisierter oder nicht funktionalisierter Alkyl-, *tert*.-Alkyl-, Benzyl- oder Allylrest ist, R' und R'', gleich oder unterschiedlich, ein Wasserstoffatom oder ein funktionalisierter oder nicht funktionalisierter Alkyl- oder *tert*.-Alkylrest sind, x eine Zahl von 1 bis 5 ist und n eine Zahl von 1 bis 25 ist.

2. Verbindung nach Anspruch 1, dadurch gekennzeichnet, daß x gleich 1 oder 2 ist und daß n eine Zahl von 1 bis 10 ist.

3. Schmiermittelzusammensetzungen, dadurch gekennzeichnet, daß sie ein Grundöl und wenigstens eine Verbindung nach Anspruch 1 oder 2 enthalten, wobei die Verbindung in einer solchen Konzentration vorhanden ist, daß sie der Zusammensetzung einen Schwefelgehalt zwischen 0,5 und 1,5 Masse% verleiht.

4. Wässerige Emulsion zur Schmierung für die Walzung metallurgischer Produkte, dadurch gekennzeichnet, daß sie ein Grundöl in einer Menge von 5 bis 12 Masse% der Emulsion und wenigstens eine Verbindung nach Anspruch 1 oder 2 enthält, wobei die Verbindung in einer solchen Konzentration vorhanden ist, daß der Schwefelgehalt der Emulsion zwischen 0,5 und 1,5 Masse% ihres Grundölgehalts beträgt.

5. Verfahren zur Synthese einer Verbindung oder eines Gemisches von Verbindungen nach Anspruch 1, dadurch gekennzeichnet, daß:
- man Polyethoxyeinheiten an eine Verbindung der Formel 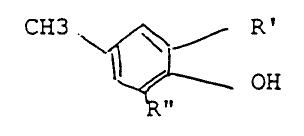 kondensiert, um eine Zwischenverbindung oder ein Gemisch von Verbindungen des Typs 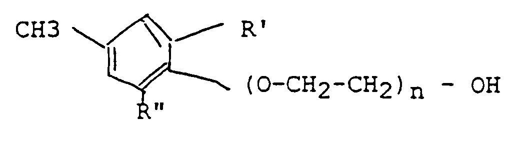 zu gewinnen;
- man die Hydroxylgruppe der Zwischenverbindung schützt;
- man das aromatische Methylen der Zwischenverbindung mit einer schwefelhaltigen Kette des Typs R-Sₓ- substituiert;
- man die Schutzgruppe der Hydroxylgruppe entfernt.

6. Verfahren zur Synthese einer Verbindung oder eines Gemisches von Verbindungen nach Anspruch 1, dadurch gekennzeichnet, daß:
- man die Hydroxylgruppe einer Verbindung des Typs 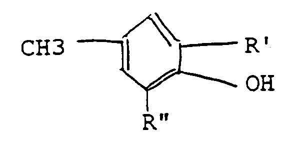 schützt;
- man das aromatische Methylen der Verbindung mit einer schwefelhaltigen Kette des Typs R-Sₓ- substituiert;
- man die Schutzgruppe der Hydroxylgruppe entfernt;
- man Polyethoxyeinheiten an die Hydroxylgruppe kondensiert.

7. Verfahren nach Anspruch 5 oder 6, dadurch gekennzeichnet, daß x gleich 1 ist und daß man die Substitution des aromatischen Methylens mit einer schwefelhaltigen Kette durchfuhrt, indem man das Methylen halogeniert und R-S⁻K⁺ an das halogenierte Methylen kondensiert.

8. Verfahren nach Anspruch 5 oder 6, dadurch gekennzeichnet, daß x gleich 2 ist und daß man die Substitution des aromatischen Methylens mit einer schwefelhaltigen Kette durchfuhrt, indem man das Methylen halogeniert, ein Bunte-Salz der so erhaltenen Verbindung herstellt und dieses Salz an die vorher hergestellte Verbindung R-S⁻K⁺ addiert.
